# EUROPÄISCHE PATENTANMELDUNG

(11) **EP 2 163 203 A1**
(43) Veröffentlichungstag der Anmeldung: **17.03.2010**
(21) Anmeldenummer: 09177620.3
(22) Anmeldetag: 24.02.2005
(51) Int. Cl.: A61B 10/00, A61B 17/20

(54) **Vorrichtung zur Durchführung eines Allergietests**

(62) Teilanmeldung aus: 05004048.4
(71) Anmelder: Schindlbeck, Gerhard, 68519 Viernheim (DE); Bachert, Claus, Prof. Dr., 47906 Kempen (DE)
(72) Erfinder: Schindlbeck, Gerhard, 68519 Viernheim (DE); Bachert, Claus, Prof. Dr., 47906 Kempen (DE)
(74) Vertreter: Müller, Gerald Christian

(57) **Zusammenfassung**

Es wird eine Vorrichtung (1) zur Durchführung eines Allergietests beschrieben, die eine Behältereinheit (2) mit mehreren Behältern (3) zur Aufnahme von Allergenen umfasst, wobei die Vorrichtung eine auf die Haut übergabefähige Kennzeichnung zur Zuordnung von bestimmten Allergenen zu bestimmten Allergieteststellen auf der Haut eines Testlebewesens aufweist. Diese Vorrichtung soll derart weitergebildet werden, dass einerseits die Allergieteststellen auf der Haut stets gut lesbar gekennzeichnet und andererseits die entsprechenden Kennzeichnungen unmittelbar nach Beendigung des Allergietest problemlos von der Haut entfernt werden können. Zur Lösung dieser Aufgabe wird vorgeschlagen, dass die übergabefähige Kennzeichnung von wenigstens einer Kennzeichnungseinheit (6) gebildet wird, die lösbar an der Vorrichtung befestigt und auf ihrer bei Durchführung des Allergietests der Haut zugewandten Seite zumindest teilweise mit einem Haftmittel versehen ist, so dass die Vorrichtung bei auf der Haut verbleibender Kennzeichnungseinheit entfernbar ist.

## Beschreibung

### I. Technisches Gebiet

Die vorliegende Erfindung bezieht sich auf eine Vorrichtung zur Durchführung eines Allergietests, bei dem die Haut eines Testlebewesens, insbesondere einer Testperson, mit Allergenen in Berührung gebracht wird und vor, nach oder während des Aufbringens der Allergene auf die Haut letztere geritzt bzw. verletzt wird, um eine Reaktion des Testlebewesens auf die Allergene hervorzurufen. Darüber hinaus bezieht sich die vorliegende Erfindung auch auf eine Vorrichtung zur Durchführung eines Allergietests, bei dem die Haut nicht geritzt bzw. verletzt werden muss.

### II. Technischer Hintergrund

Aus der DE 43 28 112 C1 ist bereits eine Vorrichtung zur Durchführung eines Allergietests der in Rede stehenden Art bekannt. Die bekannte Vorrichtung weist eine auf die Haut auflegbare Abdeckeinheit und eine mit dieser gelenkig verbundene Behältereinheit mit mehreren Behältern zur Aufnahme der Allergene auf. Nach dem Auflegen der Abdeckeinheit beispielsweise auf den Unterarm einer Testperson wird die Haut mit Hilfe eines Ritz- bzw. Prickwerkzeuges durch die Durchbrüche in der Abdeckeinheit hindurch geritzt bzw. verletzt. Anschließend kann eine die Behälter der Behältereinheit verschließende Schließfolie abgezogen und die Behältereinheit auf die Abdeckeinheit geklappt werden. Dadurch gelangen die in den Behältern befindlichen Allergene durch die Durchbrüche hindurch auf die verletzten Hautstellen, so dass die Reaktionen der Testperson auf die diversen Allergene abgewartet werden können.

Für den Fall, dass die bekannte Vorrichtung nach dem Aufbringen der Allergene auf die Haut und dem Ritzen derselben entfernt werden soll, bevor etwaige allergische Reaktionen beobachtet werden können, weist die Vorrichtung eine auf die Haut übergabefähige Kennzeichnung zur Zuordnung bestimmter Allergene zu bestimmten Allergieteststellen auf. Bei dieser übergabefähigen Kennzeichnung handelt es sich entweder um übertragbare und auf der Haut gut haftende Farbe oder um eine übertragende Folie ähnlich einem Abziehbild. Übergabefähige Kennzeichnungen dieser Art weisen den Nachteil auf, dass sie einerseits unleserlich übertragen werden können oder verwischen und andererseits in Abhängigkeit von ihrer Hauthaftung häufig länger als notwendig auf der Haut verbleiben, was aus ästhetischen Gründen von vielen Testpersonen nicht gewünscht ist.

In Zusammenhang mit der Vorrichtung gemäß DE 43 28 112 C1 ist es auch bekannt, die Behältereinheit nach dem Ritzen der Allergieteststellen auf der Haut an der Abdeckeinheit zu fixieren. Dieses Fixieren erfolgt entweder durch partielles Verkleben oder mittels eines Klettbandstreifens, der beispielsweise um den Arm der Testperson herumgeführt wird. Ein Fixieren dieser Art ist für die den Allergietest durchführende Person verhältnismäßig aufwändig, da zum Einen Hilfsmittel wie Klebstoff oder Klettbandstreifen bereitgehalten und zum Anderen mehrere Handgriffe durchgeführt werden müssen. Dieser Aufwand wirkt sich nachteilig sowohl auf die Kosten des Allergietests als auch auf die für seine Durchführung erforderliche Zeit aus.

### III. Darstellung der Erfindung

### a) Technische Aufgabe

Es ist daher die Aufgabe der vorliegenden Erfindung, eine Vorrichtung zur Durchführung eines Allergietests zu schaffen, die es ermöglicht, einerseits die Allergieteststellen auf der Haut stets gut lesbar zu kennzeichnen und andererseits die entsprechenden Kennzeichnungen unmittelbar nach Beendigung des Allergietests problemlos von der Haut entfernen zu können. Eine weitere Aufgabe der vorliegenden Erfindung besteht darin, eine Vorrichtung zur Durchführung eines Allergietests zu schaffen, die das Fixieren einer Behältereinheit auf einer Abdeckeinheit auf möglichst einfache Art und Weise erlaubt. Eine zusätzliche Aufgabe der vorliegenden Erfindung besteht darin, eine Vorrichtung zur Durchführung eines Allergietests zu schaffen, die dessen Durchführung aus der Sicht der Handhabung der Vorrichtung insgesamt vereinfacht.

### b) Lösung der Aufgabe

Diese Aufgabe wird mit einer Vorrichtung mit den Merkmalen des Anspruchs 1, 16 bzw. 24 gelöst. Weitere Ausgestaltungsformen der vorliegenden Erfindung ergeben sich aus den Unteransprüchen.

Erfindungsgemäß wird eine Vorrichtung zur Durchführung eines Allergietests vorgeschlagen, die eine Behältereinheit mit mehreren Behältern zur Aufnahme von Allergenen umfasst und bei der eine übergabefähige Kennzeichnung von wenigstens einer Kennzeichnungseinheit gebildet wird, die lösbar an der Vorrichtung befestigt ist und die auf ihrer bei Durchführung des Allergietests der Haut zugewandten Seite zumindest teilweise mit einem Haftmittel versehen ist, so dass die Vorrichtung von der Haut eines Testlebewesens entfernt werden kann und gleichzeitig die wenigstens eine Kennzeichnungseinheit auf der Haut haften bleibt. Das Trennen der Kennzeichnungseinheit von dem Rest der Vorrichtung erfolgt dabei während des Entfernens der Vorrichtung von der Haut.

In an sich bekannter Weise ist die Behältereinheit der erfindungsgemäßen Vorrichtung vorzugsweise flach ausgebildet und besteht ebenso vorzugsweise aus einem dünnen, flexiblen Material, wie beispielsweise Kunststoff, das an die Kontur der Allergieteststelle, wie etwa dem Unterarm einer Testperson, anpassbar ist. Die Kennzeichnungseinheit kann aus demselben Material wie die Behältereinheit bestehen und beispielsweise entlang einer perforierten Reißlinie an einer Seitenkante der Behältereinheit befestigt sein. Dies bringt den Vorteil mit sich, dass Behältereinheit und Kennzeichnungseinheit in ein und demselben Fertigungsschritt hergestellt werden können. Nach dem Entfernen der Behältereinheit von der Allergieteststelle verbleibt die wenigstens eine Kennzeichnungseinheit ähnlich wie ein Klebestreifen auf der Haut der Testperson, so dass anhand der Informationen, die auf der von der Haut abgewandten Seite der Kennzeichnungseinheit aufgebracht sind, eine eindeutige Zuordnung eines bestimmten Allergens zu einer bestimmten Allergieteststelle zum Zwecke der Auswertung des Allergietests erfolgen kann. Dementsprechend werden die Zuordnungsinformationen nicht mehr mittels Farbe oder in der Art eines Abziehbildes direkt auf die Haut aufgebracht. Die in der Art eines Klebestreifens ausgebildete Kennzeichnungseinheit haftet lediglich auf der Haut des Testlebewesens und kann nach Abschluss der Allergietestauswertung problemlos von der Haut abgezogen werden.

Eine Kennzeichnungseinheit im Sinne der vorliegenden Erfindung kann auch bei solchen Vorrichtungen Anwendung finden, die zusätzlich zu der Behältereinheit eine Abdeckeinheit mit mehreren Durchbrüchen zur Abgrenzung der Allergieteststellen aufweist. Dabei ist die Abdeckeinheit in an sich bekannter Weise auf die Haut des Testlebewesens auflegbar und die Behältereinheit ist ihrerseits derart auf die Abdeckeinheit auflegbar, dass die Durchbrüche mit den Behältern im Wesentlichen fluchten. Die Kennzeichnungseinheit kann bei Vorrichtungen dieser Art entweder an einer Seitenkante der Behältereinheit oder an einer Seitenkante der Abdeckeinheit lösbar befestigt sein. Da Behältereinheit und Abdeckeinheit in der Aufsicht vorzugsweise dieselbe Geometrie aufweisen, ragt die Kennzeichnungseinheit in Abhängigkeit davon, ob sie an der Abdeckeinheit oder an der Behältereinheit befestigt ist, seitlich über die Behältereinheit oder die Abdeckeinheit hinaus. Bei an der Behältereinheit angeordneter Kennzeichnungseinheit wird der besondere Vorteil erreicht, dass die erfindungsgemäße Vorrichtung aufgrund der klebebandartigen Niederhaltewirkung der Kennzeichnungseinheit in durchgehend flächigem Kontakt auf der meist gekrümmten Hautoberfläche der Allergieteststelle aufliegt.

Die erfindungsgemäße Kennzeichnungseinheit liegt in ihrer von der Behältereinheit oder der Abdeckeinheit abragenden Stellung vorzugsweise in der von der Behältereinheit oder der Abdeckeinheit aufgespannten Ebene. Zur Minimierung der Größe der erfindungsgemäßen Vorrichtung und somit des Platzbedarfs während Transport und Lagerung ist es denkbar, die Kennzeichnungseinheit zunächst in einem auf die Behältereinheit oder auf die Abdeckeinheit geklappten Zustand anzuordnen. Soll die Vorrichtung schließlich zur Durchführung eines Allergietests verwendet werden, so wird die Kennzeichnungseinheit um im Wesentlichen 180° um diejenige Seitenkante, an der sie befestigt ist, in ihre von der Behältereinheit oder der Abdeckeinheit abragende Stellung geschwenkt bzw. geklappt.

Insbesondere wenn in an sich bekannter Weise mehrere im Wesentlichen parallele Reihen von Behältern oder von Behältern und Durchbrüchen vorgesehen sind, ist vorzugsweise an zwei gegenüber liegenden Seitenkanten der Behältereinheit oder der ggf. vorhanden Abdeckeinheit jeweils eine Kennzeichnungseinheit angeordnet. Die Zuordnungsinformationen der jeweiligen Kennzeichnungseinheit betreffen dann diejenige Reihe von Behältern bzw. Durchbrüchen bzw. Allergieteststellen, die der Kennzeichnungseinheit am Nächsten liegt.

Denkbar ist auch, nicht nur die Kennzeichnungseinheit lösbar an der Behältereinheit oder der Abdeckeinheit anzuordnen, sondern zusätzlich die Behälter aus der Behältereinheit herauslösbar auszubilden. Bei dieser Variante kann die Behältereinheit auf die Haut aufgelegt bzw. aufgeklebt und anschließend die Haut mit Hilfe eines Prickwerkzeuges durch die Behälter hindurch geritzt werden. Das Abziehen der Behältereinheit von der Haut erfolgt derart, dass sowohl die einzelnen Behälter der Behältereinheit als auch die Kennzeichnungseinheit auf der Haut haften bleiben. Nach einer gewissen Einwirkdauer können schließlich die einzelnen Behälter entfernt und die Auswertung des Allergietests mit Hilfe der Zuordnungsinformationen auf der Kennzeichnungseinheit durchgeführt werden.

Die erfindungsgemäße Vorrichtung kann auch ein Rahmenteil mit einer Rahmenöffnung aufweisen, die bei auf die Haut aufgelegter Vorrichtung das Arbeitsfeld, in dem sich die Allergieteststellen befinden, definiert. Dabei sind die Behältereinheit, die Abdeckeinheit und die wenigstens eine Kennzeichnungseinheit jeweils an einer äußeren Seitenkante des Rahmenteils befestigt. Insbesondere zur Minimierung der Größe der Vorrichtung und somit des Platzbedarfs bei Transport und Lagerung können die Behältereinheit und die Abdeckeinheit gelenkig an dem Rahmenteil befestigt sein, so dass sie um im Wesentlichen 180° in die Rahmenöffnung klapp- bzw. schwenkbar sind.

Wie bei der Variante ohne Rahmenteil kann die Kennzeichnungseinheit auch bei der Variante mit Rahmenteil entlang einer perforierten Reißlinie lösbar befestigt sein, allerdings nicht an der Behältereinheit und nicht an der Abdeckeinheit, sondern an dem Rahmenteil. Die Behältereinheit und die Abdeckeinheit sind vorzugsweise an gegenüberliegenden Seiten des Rahmenteils befestigt. Die Kennzeichnungseinheit kann um im Wesentlichen 180° zwischen einer von der Seitenkante des Rahmenteils abragenden Stellung in eine im Wesentlichen in der Rahmenöffnung liegende Stellung verschwenkt werden. Insbesondere bei im Wesentlichen parallelen Reihen von Behältern und Durchbrüchen befindet sich die Kenzeichnungseinheit vorzugsweise an zwei gegenüberliegenden Seitenkanten des Rahmenteils.

Bei dem auf der der Haut zugewandten Seite der Kennzeichnungseinheit angebrachten Haftmittel kann es sich um einen Klebefilm handeln, der zum Schutz von Verunreinigungen mit einer entfernbaren Schutzfolie bedeckt ist. Vorzugsweise ist die gesamte der Haut zugewandte Seite der Kennzeichnungseinheit mit dem Klebefilm überzogen. Denkbar ist jedoch auch ein nur teil- oder stellenweiser Überzug, solange ein sicheres Haften auf der Haut des Testlebewesens gewährleistet ist.

Erfindungsgemäß wird auch eine Vorrichtung zur Durchführung eines Allergietests vorgeschlagen, die eine Behältereinheit mit mehreren Behältern zur Aufnahme von Allergenen und eine Abdeckeinheit mit mehreren Durchbrüchen zur Abgrenzung von Allergieteststellen umfasst und bei der auf derjenigen Seite der Abdeckeinheit, auf die die Behältereinheit auflegbar ist, zumindest teilweise ein Klebefilm vorgesehen ist, der von einer Abziehfolie bedeckt ist, die mit den Durchbrüchen in der Abdeckeinheit im Wesentlichen fluchtende Durchgangslöcher aufweist. Der Vorteil einer derartigen Vorrichtung besteht darin, dass durch einen einzigen Handhabungsschritt, nämlich das Entfernen der Abziehfolie, gewährleistet ist, dass die Behältereinheit beim Auflegen auf die Abdeckeinheit sicher und unverrutschbar auf letzterer fixiert wird. Ein Verlaufen oder Verwischen von Allergenen zwischen Abdeckeinheit und Behältereinheit wird vermieden. Darüber hinaus können Behältereinheit und Abdeckeinheit nach ausreichender Einwirkdauer als eine zusammenklebende, kompakte Einheit von der Allergieteststelle entfernt und entsorgt werden.

Vorzugsweise liegen die Querschnitte der Durchgangslöcher in der Abziehfolie vollständig innerhalb der Querschnitte der Durchbrüche in der Abdeckeinheit. Des weiteren können die Durchgangslöcher und die Durchbrüche dieselbe Querschnittsform und denselben Querschnittsmittelpunkt aufweisen. Sind dann zusätzlich noch die Querschnittsflächen der Durchgangslöcher kleiner als die Querschnittsflächen der Durchbrüche, so ergibt sich der Vorteil, dass die Haut beim Ritzen mit einem Prickwerkzeug durch die Durchgangslöcher und die Durchbrüche hindurch immer in einem Hautbereich geritzt wird, der bezüglich der Durchgangslöcher in der Abdeckeinheit zentriert liegt. Dadurch wird das Überlappen allergischer Reaktionen bei benachbarten Allergieteststellen unwahrscheinlicher.

Die Querschnitte der Durchgangslöcher und/oder der Durchbrüche können beispielsweise kreisförmig, rechteckförmig oder quadratisch ausgebildet werden. Dabei müssen Durchgangslöcher und Durchbrüche nicht zwingend identische Querschnittsform aufweisen. Je nach Bedarf sind auch Kombinationen verschiedener Querschnittsgeometrien denkbar. Vorteilhaft ist es, die Durchgangslöcher in der Abziehfolie kreuzförmig auszubilden. Dies ermöglicht ein kreuzweises Ritzen der Allergieteststellen auf der Haut, so dass die Haut zumindest in dem Kreuzmittelpunkt zuverlässig geritzt wird.

In besonders vorteilhafter Weise können die Allergieteststellen voneinander abgedichtet bzw. isoliert werden, wenn jeder Behälter der Behältereinheit in seinem Randbereich eine Dichtlippe aufweist. Vorzugsweise durchgreifen die Dichtlippen bei auf die Abdeckeinheit aufgelegter Behältereinheit den jeweiligen Durchbruch, so dass die Dichtlippen jeweils in Kontakt mit der Haut um die jeweilige Allergieteststelle herum gebracht werden können. Dadurch werden die Allergieteststellen wirksam voneinander abgedichtet, so dass ein einer Teststelle zugeordnetes Allergen nicht zu einer anderen Allergieteststelle gelangen kann. Die erfindungsgemäße Dichtlippe kann in besonders vorteilhafter Weise beispielsweise beim Heißsiegelverkleben einer die Behälter verschließenden Schließfolie mit der Behältereinheit erzeugt werden.

Erfindungsgemäß wird außerdem eine Vorrichtung zur Durchführung eines Allergietests vorgeschlagen, bei der die Behälter einer Behältereinheit sowohl auf ihrer bei Durchführung des Allergietests der Haut zugewandten Seite als auch auf ihrer bei Durchführung des Allergietests von der Haut abgewandten Seite mit einem vorzugsweise folienartigen Material verschlossen sind, das mittels eines Prickwerkzeugs durchstechbar ist, so dass durch Durchstechen eines Behälters der auf der Haut aufliegenden Behältereinheit einerseits eine Pricknadel des Prickwerkzeugs mit Allergen benetzbar und andererseits die Haut verletzbar sowie dadurch das Allergen in die Haut einbringbar ist.

Eine Vorrichtung dieser Art kann in vorteilhafter Weise ohne das Auflegen der Behältereinheit auf eine Abdeckeinheit sowie ohne einen separaten Arbeitsschritt zum Öffnen der Behälter gehandhabt werden. Eine Kennzeichnungseinheit der voranstehend erläuterten Art kann bei Bedarf vorgesehen werden. Alternativ besteht die Möglichkeit, in an sich bekannter Weise auf der bei Durchführung des Allergietests der Haut zugewandten Seite der Behältereinheit in der Art eines Abziehbildes auf die Haut übertragbare Zuordnungsinformationen zur Zuordnung bestimmter Allergene zu bestimmten Allergieteststellen vorzusehen.

Besonders vorteilhaft ist es, wenn bei einer Vorrichtung dieser Art die Behälter auf ihrer bei Durchführung des Allergietests von der Haut abgewandten Seite jeweils einen kamin- oder rohrstutzenartigen Fortsatz aufweisen, der das Prickwerkzeug beim Durchstechen des Behälters führt. Vorzugsweise entspricht dabei die Innenquerschnittsform des Fortsatzes im Wesentlichen derjenigen Querschnittsform des Bereichs des Prickwerkzeugs, der geführt werden soll. Die Führung des Prickwerkzeugs erfolgt zwecks freier Beweglichkeit desselben mit einem gewissen Spiel.

### c) Ausführungsbeispiele

Nachfolgend werden mehrere Ausführungsformen der vorliegenden Erfindung beispielhaft anhand der beigefügten Zeichnungen beschrieben. Es zeigen:
- Fig. 1:: eine perspektivische Ansicht einer ersten Ausführungsform der erfindungsgemäßen Vorrichtung;
- Fig. 2:: eine Schnittansicht gemäß Schnitt A-A in Fig. 1;
- Fig. 3:: eine Schnittansicht gemäß Schnitt B-B in Fig. 1;
- Fig. 4:: eine Aufsicht auf die Vorderseite der Vorrichtung gemäß Fig. 1 ohne Schließ- und Abziehfolie;
- Fig. 5:: eine Aufsicht auf die Rückseite der Vorrichtung gemäß Fig. 1;
- Fig. 6:: eine Aufsicht auf die der Haut abgewandten Seite einer zweiten Ausführungsform der erfindungsgemäßen Vorrichtung; und
- Fig. 7:: eine Aufsicht auf eine dritte Ausführungsform der erfindungsgemäßen Vorrichtung.

In den Zeichnungen sind gleiche Teile, die gleiche Funktionen haben, mit denselben Bezugszeichen gekennzeichnet.

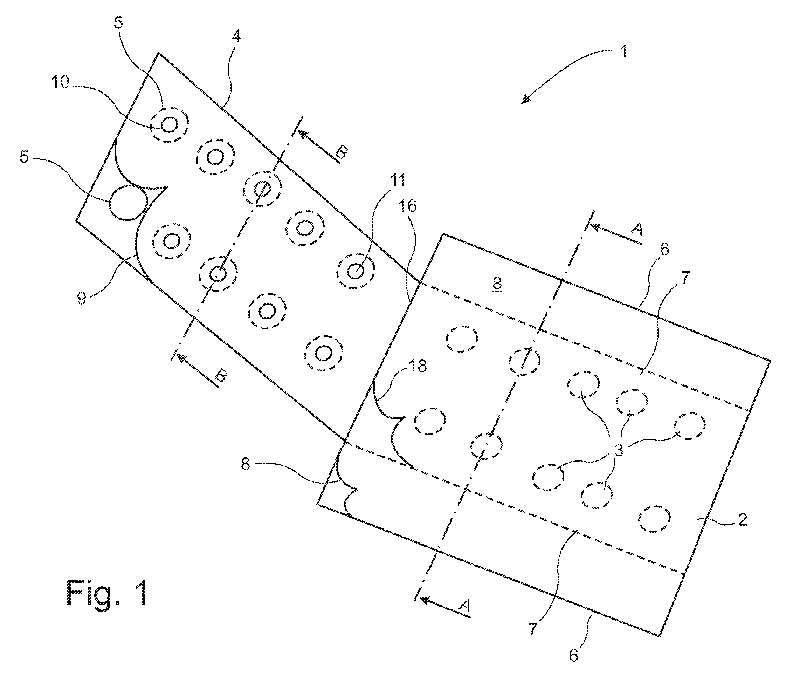

In Fig. 1 ist eine erste Ausführungsform der erfindungsgemäßen Vorrichtung 1 dargestellt. Die Vorrichtung 1 besteht aus einer flachen, rechteckförmigen, als Kammerteil fungierenden Behältereinheit 2, einer flachen, rechteckförmigen, als Schablonenteil fungierenden Abdeckeinheit 4 sowie zwei flachen, rechteckförmigen Kennzeichnungseinheiten 6, die auf den gegenüber liegenden langen Seiten der Abdeckeinheit 4 flügelartig angeordnet sind. Die Behältereinheit 2, die Abdeckeinheit 4 und die Kennzeichnungseinheiten 6 sind flexibel und biegsam ausgestaltet, so dass sie an Krümmungen oder Wölbungen der Hautoberfläche angepasst werden können. Bei der gezeigten Ausführungsform bestehen die vorgenannten Einheiten aus einem dünnen Kunststoff, der ggf. in geeigneter Weise beschichtet ist.

In an sich bekannter Weise ist die Behältereinheit 2 mit Hilfe eines Gelenks 16 klapp- oder drehbar mit der Abdeckeinheit 4 verbunden. Bei auf die Abdeckeinheit 4 geklappter Behältereinheit 2 ragen somit die beiden Kennzeichnungseinheiten 6 über die langen Seiten der Abdeckeinheit 4 hinaus und können in Kontakt mit der Haut gebracht werden. Die Kennzeichnungseinheiten 6 sind jeweils lösbar entlang einer perforierten Reißlinie 7 an der Behältereinheit 2 befestigt. Alternativ kann die Lösbarkeit durch eine Materialverdünnung zwischen Kennzeichnungseinheiten 6 und Behältereinheit 2 bewirkt werden. Zusätzlich können die Reißlinien 7 als Gelenke fungieren, so dass die Kennzeichnungseinheiten 6 von der in den Fig. 1, 4 und 5 gezeigten Stellung um ca. 180° auf die Behältereinheit 2 geklappt werden können. Dabei ist ein Klappen auf die in Fig. 1 sichtbare Vorderseite oder auf die in Fig. 1 nicht sichtbare Rückseite der Behältereinheit 2 denkbar. Vorzugsweise erfolgt das Einklappen der Kennzeichnungseinheiten 6 auf die in Fig. 1 gezeigte Vorderseite, da in diesem Fall die Kennzeichnungseinheiten 6 bei auf die Abdeckeinheit 4 geklappter Behältereinheit 2 zwischen Abdeckeinheit 4 und Behältereinheit 2 verstaut werden können. Unabhängig von der Klapprichtung der Kennzeichnungseinheiten 6 wird durch das Einklappen derselben ein Platzspareffekt bewirkt, der dazu führt, dass die erfindungsgemäße Vorrichtung 1 nicht mehr Platz benötigt als die aus dem Stand der Technik bekannten Vorrichtungen zur Durchführung von Allergietests.

Die Behältereinheit 2 weist in an sich bekannter Weise Behälter 3 zur Aufnahme von Allergenen auf. Wie in den Fig. 1, 4 und 5 zu erkennen ist, sind insgesamt zwei parallele Reihen mit jeweils fünf Behältern 3 vorgesehen. Denkbar sind jedoch auch beliebige andere Geometrien von Behälteranordnungen, sofern sie den Erfordernissen der Durchführung des Allergietests Rechnung tragen. Beispiele hierfür sind Behälteranordnungen, deren Geometrien in der Aufsicht Tier- bzw. Comicfigurenumrisse oder eine Herzform widerspiegeln. Behälteranordnungen dieser Art können Kindern ggf. eine etwaige Angst vor dem Allergietest nehmen. Außerdem können im Übrigen beliebige Formen, Farben und Anzahlen von in der Behältereinheit 2 ausgebildeten Behältern 3 vorgesehen werden.

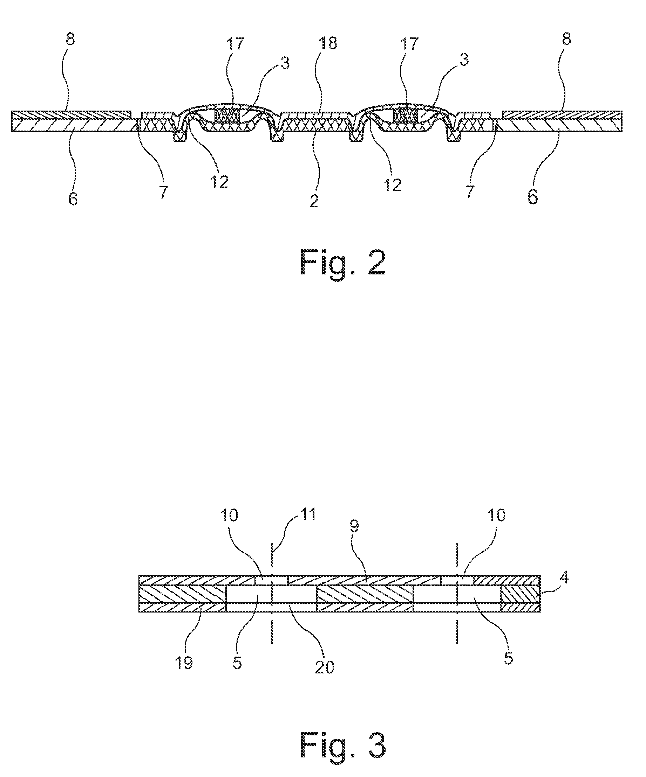

Fig. 2 zeigt die Schnittansicht gemäß Schnitt A-A in Fig. 1 durch zwei Behälter 3. Bei der gezeigten Ausführungsform befindet sich in den Behältern 3 jeweils ein mit einem bestimmten Allergen getränktes vliesartiges Schwammgewebe 17. Anstatt in flüssiger Form können die Allergene allerdings auch ohne Schwammgewebe 17 in fester, gelartiger oder pastenartiger Form in den Behältern 3 angeordnet sein. Darüber hinaus ist alternativ denkbar, in den Behältern 3 eine Pricknadel, an der sich ein Allergen befindet, anzuordnen. Ein Verletzen der Haut ist dann durch Drücken auf den jeweiligen Behälter 3 von der in Fig. 2 unteren Seite auf die Behältereinheit möglich. Die Behälter 3 sind bei der gezeigten Ausführungsform mittels einer vorzugsweise aus Aluminium bestehenden Schließfolie 18 verschlossen. Die Schließfolie 18 sorgt dafür, dass die in den Schwammgeweben 17 enthaltenen Allergene haltbar bleiben und möglichst lange für die Zwecke eines Allergietests verwendet werden können. Zum Öffnen der Behälter 3 kann die Folie von Hand von der Behältereinheit 2 abgezogen werden. Zu diesem Zweck kann die Schließfolie 18 eine Grundfläche aufweisen, die geringfügig größer als diejenige der Behältereinheit 2 ist. Der über die Seitenränder der Behältereinheit 2 hinaus stehende Teil der Schließfolie 18 kann dann als Lasche zum Abziehen der Schutzfolie 18 verwendet werden.

In Fig. 2 sind auch die beiden Kennzeichnungseinheiten 6 zu erkennen, die über die perforierte Reißlinie 7 mit der Behältereinheit 2 verbunden sind. Auf der in Fig. 2 oberen Seite der Kennzeichnungseinheiten 6 befindet sich jeweils eine abziehbare Schutzfolie 8, die einen auf der in Fig. 2 oberen Seite der Kennzeichnungseinheiten 6 jeweils befindlichen Klebefilm abdeckt. Dieser Klebefilm ermöglicht ein Fixieren der Kennzeichnungseinheiten 6 auf der Haut eines Testlebewesens, insbesondere am Unterarm einer Testperson.

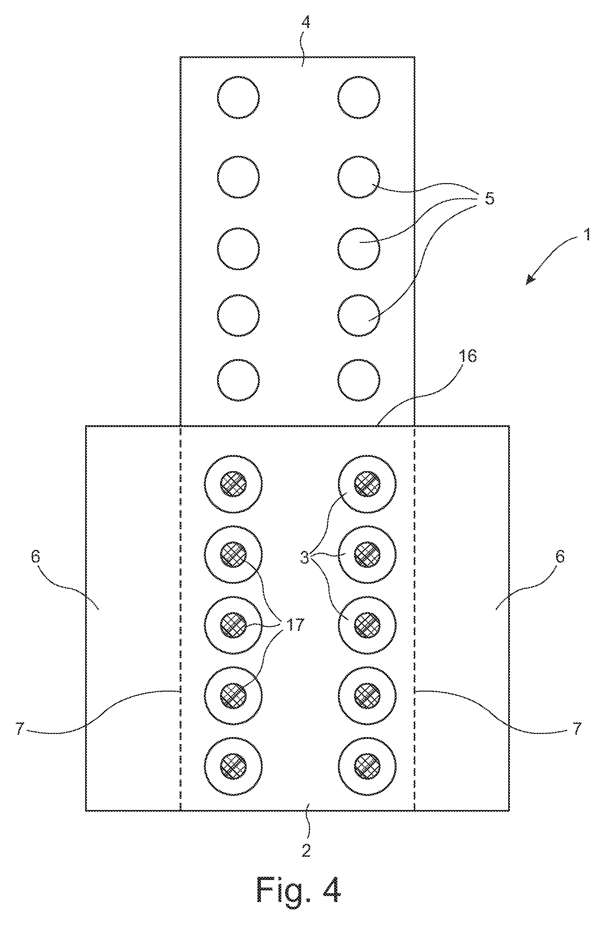

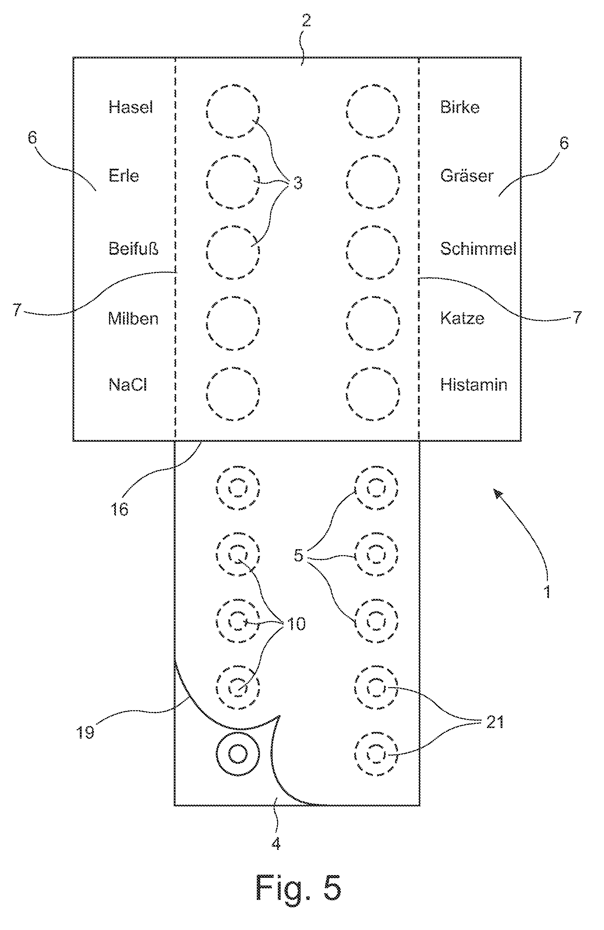

Auf der in Fig. 2 unteren Seite der Kennzeichnungseinheiten 6, d.h. auf der in den Fig. 1 und 4 nicht sichtbaren Rückseite, die in Fig. 5 zu sehen ist, befinden sich erfindungsgemäß jeweils Zuordnungsinformationen, die Auskunft darüber geben, welche Art von Allergen in einem bestimmten Behälter 3 enthalten ist. Wie in Fig. 5 zu erkennen, sind die Zuordnungsinformationen zu diesem Zweck derart auf die Kennzeichnungseinheiten 6 aufgebracht, dass sie sich unmittelbar neben dem jeweiligen Behälter 3 befinden, dessen Allergen sie kennzeichnen sollen. Bei dem gezeigten Ausführungsbeispiel sind die Zuordnungsinformationen jeweils als Wort aufgebracht, das die Allergie wiedergibt, auf die das in dem jeweiligen Behälter 3 befindliche Allergen die Testperson testet. Wie in Fig. 5 zu erkennen, handelt es sich um die Worte "Hasel" "Erie", "Beifuß", "Milben", "NaCl", "Birke", "Gräser", "Schimmel", "Katze" und "Histamin". Die Zuordnungsinformationen können im Rahmen der vorliegenden Erfindung natürlich auch von anderen Allergenangaben in Text- und/oder Symbolform gebildet werden.

Fig. 3 zeigt den Schnitt B-B gemäß Fig. 1 durch die Abdeckeinheit 4. Die Abdeckeinheit 4 weist Durchbrüche 5 auf, die bei auf die Abdeckeinheit 4 geklappter Behältereinheit 2 in an sich bekannter Weise mit den Behältern 3 im Wesentlichen fluchten. Auf der in Fig. 3 oberen Seite der Abdeckeinheit 4, auf die auch in Fig. 1 geblickt wird, befindet sich eine Abziehfolie 9, die einen auf der in Fig. 3 oberen Seite der Abdeckeinheit 4 befindlichen Klebefilm bedeckt. Dieser Klebefilm kann im Wesentlichen auf der gesamten oder nur auf einem Teil der Oberfläche der Abdeckeinheit 4 aufgebracht sein. Beispielsweise ist denkbar, dass nur einige Klebefilmpunkte vorgesehen sind. Der von der Abziehfolie 9 bedeckte Klebefilm ermöglicht ein sicheres Fixieren der auf die Abdeckeinheit 4 geklappten Behältereinheit 2 an bzw. auf der Abdeckeinheit 4.

Darüber hinaus weist die Abziehfolie 9 Durchgangslöcher 10 auf, die jeweils mit den Durchbrüchen 5 in der Abdeckeinheit 4 fluchten und dabei - in Fig. 3 von oben betrachtet - mit ihrer Querschnittsform vollständig innerhalb der Querschnittsform des jeweiligen Durchbruchs 5 liegen. Der besondere Vorteil dieser Ausgestaltung wird in Zusammenhang mit der Funktionsweise der Vorrichtung 1 weiter unten beschrieben. Bei der gezeigten Ausführungsform weisen sowohl die Durchbrüche 5 als auch die Durchgangslöcher 10 eine kreisrunde Geometrie auf, wobei die jeweiligen Kreismittelpunkte 11 aufeinander fallen. Denkbar sind hier auch andere Querschnittsgeometrien, wie beispielsweise quadratische, rechteckige, elliptische oder kreuzförmige Formen. Auch voneinander abweichende Querschnittsgeometrien für die Durchbrüche 5 einerseits und die Durchgangslöcher 10 andererseits sind denkbar.

Auf der in Fig. 3 unteren Seite der Abdeckeinheit 4 ist eine weitere Abziehfolie 19 angebracht, die einen weiteren, auf der in Fig. 3 unteren Seite der Abdeckeinheit 4 befindlichen Klebefilm bedeckt. Dieser zumindest teilweise aufgebrachte Klebefilm ermöglicht das sichere Befestigen bzw. Fixieren der Abdeckeinheit 4 auf der Hautoberfläche der Testperson. Die Abziehfolie 19 weist ihrerseits Durchgangsöffnungen 20 auf, die bei der gezeigten Ausführungsform nach Anzahl, Lage, Querschnittsgeometrie und -größe den Durchbrüchen 5 entsprechen. Denkbar ist auch, die Durchgangsöffnungen 20 mit anderen Querschnittsgeometrien und -größen auszubilden oder die Abziehfolie 19 vollständig ohne Durchgangsöffnungen 20 auszubilden, da die Abziehfolie 19 vor Durchführung des eigentlichen Allergietests, insbesondere des Hautritzens, vollständig von der Abdeckeinheit 4 entfernt wird.

Fig. 4 zeigt eine Aufsicht auf die in Fig. 1 zu erkennende Vorderseite der erfindungsgemäßen Vorrichtung 1, wobei von der Behältereinheit 3 die Schließfolie 18, von den Kennzeichnungseinheiten 6 die Schutzfolien 8 und von der Abdeckeinheit 4 die Abziehfolie 9 entfernt wurden. Dementsprechend sind in Fig. 4 die offenen Behälter 3 mit den darin befindlichen Schwammgeweben 17 zu erkennen. Des weiteren sind in der Abdeckeinheit 4 die Durchbrüche 5 zu erkennen, deren äußere Randbereiche nicht mehr von der Abziehfolie 9 bedeckt sind.

Fig. 5 zeigt eine Ansicht auf die in den Fig. 1 und 4 nicht sichtbare Rückseite der erfindungsgemäßen Vorrichtung 1. Wie in Fig. 4 liegen auch hier die Behältereinheit 2 und die Abdeckeinheit 4 in einer Ebene. Die in der Behältereinheit 2 angeordneten Behälter 3 sind in Fig. 5 nur von ihrer verschlossenen Rückseite her sichtbar. Auf den beiden Kennzeichnungseinheiten 6 sind die Zuordnungsinformationen "Hasel", "Erle" "Beifuß", "Milben", "NaCl", "Birke", "Gräser", "Schimmel", "Katze" und "Histamin" sichtbar. Durch die in der Abdeckeinheit 4 vorgesehenen Durchbrüche 5 hindurch sind kreisringförmige Bereiche 21 der Abziehfolie 9 sichtbar.

Nachfolgend wird nun beispielhaft die Funktions- und Anwendungsweise der Vorrichtung 1 gemäß der Fig. 1-5 im Rahmen der Durchführung eines Allergietests beschrieben:

Nach Vorbereitung einer Hautoberfläche einer Testperson, beispielsweise am Unterarm, durch Reinigen derselben wird die Abziehfolie 19 von der Abdeckeinheit 4 entfernt und letztere mit Hilfe des freigelegten Klebefilms durch Andrücken auf der Hautoberfläche befestigt. Anschließend wird mit Hilfe eines an sich bekannten Prick- oder Ritzwerkzeugs die Hautoberfläche durch die in der Abziehfolie 9 befindlichen Durchgangslöcher 10 hindurch geritzt. Hierzu kann mit dem Prickwerkzeug beispielsweise jeweils einmal geradlinig und ohne Absetzen über die in Fig. 1 gezeigten Reihen von Durchgangslöchern 10 gefahren werden. Nach dem Ritzen der Hautoberfläche wird die Abziehfolie 9 entfernt, um den darunter liegenden Klebefilm freizulegen. Des Weiteren werden die Behälter 3 durch Abziehen der Schließfolie 18 geöffnet und die Klebefilme auf den Kennzeichnungseinheiten 6 durch Entfernen der Schutzfolien 8 freigelegt. Daraufhin kann die Behältereinheit 2 samt Kennzeichnungseinheiten 6 um ca. 180° um das Gelenk 16 derart geklappt werden, dass die Behälter 3 mit den Durchbrüchen 5 im Wesentlichen fluchten und die Allergene in den Schwammgeweben 17 auf die Allergieteststellen gelangen, die von den Durchbrüchen 5 auf der Hautoberfläche abgegrenzt werden.

Aufgrund des Klebefilms auf der von der Haut abgewandten Seite der Abdeckeinheit 4 haftet die Behältereinheit sicher und unverrutschbar auf der Abdeckeinheit 4. Behältereinheit 2 und Abdeckeinheit 4 bilden eine kompakt handhabbare Einheit, die nicht mehr nur linienförmig über das Gelenk 16, sondern flächig miteinander verbunden ist. Darüber hinaus werden die beiden Kennzeichnungseinheiten 6 auf dem Unterarm der Testperson festgedrückt, so dass sie an der Hautoberfläche haften.

Nach einer gewissen Einwirkdauer auf die Allergieteststellen kann die kompakt handhabbare Einheit aus Behältereinheit 2 und Abdeckeinheit 4 von der Hautoberfläche entfernt werden, indem sie ergriffen und bei gleichzeitigem Andrücken bzw. Festhalten der Kennzeichnungseinheiten 6 an der Hautoberfläche entfernt wird. Dabei wird die Behältereinheit 2 entlang der perforierten Reißlinien 7 von den Kennzeichnungseinheiten 6 abgerissen, die zur Auswertung des Allergietests auf der Hautoberfläche der Testperson verbleiben. Die Reaktionen der Testperson auf die einzelnen Allergene kann dann von der den Test durchführenden Person abgelesen und mit Hilfe der auf den Kennzeichnungseinheiten 6 befindlichen Zuordnungsinformationen dokumentiert werden. Nach Beendigung der Dokumentation des Testergebnisses können die Kennzeichnungseinheiten 6 von der Hautoberfläche abgezogen werden, so dass keine ggf. erst nach längerer Zeit vollständig entfernbaren Kennzeichnungen mehr am Unterarm der Testperson zurückbleiben. Bei Bedarf können sogar die Kennzeichnungseinheiten 6 durch Einkleben oder sonstiges Ablegen in einer ärztlichen Akte im Rahmen der Dokumentation verwendet werden.

Beim Ritzen der Haut mit dem Prickwerkzeug bringen die in der Abziehfolie 9 vorhandenen Durchgangslöcher 10 einen besonderen Vorteil mit sich. Die Ritzung der Haut erfolgt im Hinblick auf die größeren Durchbrüche 5 im Wesentlichen zentriert. Dadurch werden Hautritzungen am Rand der Durchbrüche 5 vermieden, so dass ein unerwünschtes Annähern zweier geritzter Allergieteststellen an einander unmöglich ist. In besonders vorteilhafter Weise kann auch die Zuverlässigkeit der Hautritzung gesteigert werden. Hierzu ist denkbar, nicht kreisrunde Durchgangslöcher 10, sondern kreuzförmige Durchgangslöcher 10 auszubilden. Das Ritzen der Haut kann dann erfolgen, indem das Prickwerkzeug in zwei im Wesentlichen senkrecht zueinander verlaufenden Ritzgeraden über das jeweils kreuzförmige Durchgangsloch 10 geführt wird. Zumindest im Schnittpunkt der beiden Ritzgeraden wird die Hautoberfläche zuverlässig geritzt.

Um zuverlässig auszuschließen, dass das einer bestimmten Allergieteststelle zugeordnete Allergen zwischen Hautoberfläche und Abdeckeinheit 4 zu einer anderen Allergieteststelle gelangt, was das Ergebnis des Allergietests naturgemäß verfälschen würde, weist die Behältereinheit 2 eine in Fig. 2 zu erkennende Dichtlippe 12 auf. Die Dichtlippe 12 liegt im Randbereich eines jeden Behälters 3 und bildet vorzugsweise zusätzlich dessen Seitenwandung. Sie kann in der Aufsicht entweder dieselbe Geometrie aufweisen wie die Durchbrüche 5 in der Abdeckeinheit 4 oder bei Bedarf mit einer davon abweichenden Geometrie ausgebildet sein. Wie in Fig. 2 zu erkennen ist, steht die Dichtlippe 12 in Fig. 2 nach oben über die Oberfläche der Behältereinheit 2 hervor, so dass sie beim Zusammenlegen von Behältereinheit 2 und Abdeckeinheit 4 durch die in letzterer vorhandenen Durchbrüche 5 hindurch greift und in Kontakt mit der Hautoberfläche treten kann. Dadurch wird die jeweilige Allergieteststelle durch Berührung zwischen Dichtlippe 12 und Hautoberfläche von anderen Allergieteststellen sicher isoliert. Bei der gezeigten Ausführungsform wird die Dichtlippe 12 in besonders vorteilhafter Weise gleichzeitig mit dem Heißsiegelverkleben der Schließfolie 18 an der Behältereinheit 2 hergestellt. Dabei wird in Fig. 2 von oben ein Heißsiegelstempel mit hier kreisförmiger Stempelfläche an die Schließfolie 18 herangeführt, der letztere mit der Behältereinheit 2 verpresst. Dabei ergeben sich Materialverdrängungen insbesondere an der Behältereinheit 2, die zur Ausbildung der in Fig. 2 gezeigten Dichtlippe 12 führen.

Im Sinne der vorliegenden Erfindung ist es alternativ möglich, die Kennzeichnungseinheiten 6 nicht an der Behältereinheit 2, sondern stattdessen an den langen Seiten der Abdeckeinheit 4 anzuordnen. Die voranstehend beschriebene Funktionsweise der Vorrichtung 1 ändert sich dadurch im Wesentlichen nicht. Vor dem Auflegen der Abdeckeinheit 4 auf die Hautoberfläche sind zusätzlich zu der Abziehfolie 19 lediglich noch die Schutzfolien 8 von den Kennzeichnungseinheiten 6 abzuziehen.

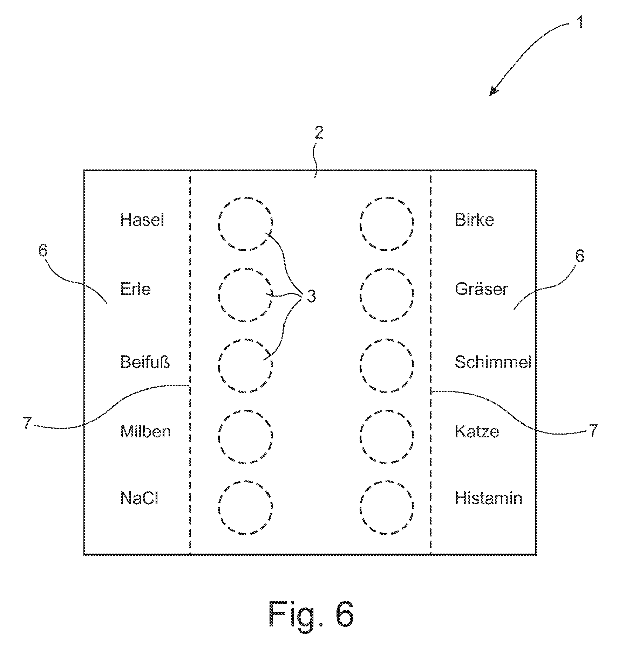

Fig. 6 zeigt eine zweite Ausführungsform einer erfindungsgemäßen Vorrichtung 1, wobei die Blickrichtung derjenigen der Fig. 5 entspricht, d.h. die Rückseite der Vorrichtung 1 gezeigt ist. Bei dieser Ausführungsform wurde ersatzlos auf die bei der ersten Ausführungsform vorhandene Abdeckeinheit 4 verzichtet, wobei sie ansonsten unverändert der ersten Ausführungsform entspricht. Die Vorrichtung 1 ermöglicht erfindungsgemäß die Durchführung eines Allergietests ohne das von manchen Testpersonen nicht geschätzte Ritzen oder Pricken der Haut. Zu diesem Zweck enthält das in den Behältern 3 befindliche Schwammgewebe 17 nicht nur das jeweilige Allergen, sondern zusätzlich einen die Hautschranke chemisch und/oder biologisch und/oder physikalisch öffnenden Stoff. Ein Beispiel für einen derartigen Stoff ist Dimethylsulfoxid. Das Ritzen oder Pricken der Haut wird in diesem Fall durch das Öffnen der Hautschranke während der Einwirkdauer nach dem Auflegen der Behältereinheit 2 auf die Hautoberfläche ersetzt. Die Kennzeichnungseinheiten 6 dienen in derselben Weise wie in Zusammenhang mit der ersten Ausführungsform beschrieben zur Zuordnung bestimmter Allergene zu bestimmten Allergieteststellen.

Bei der Ausführungsform gemäß Fig. 6 besteht allerdings auch die Möglichkeit, den Allergietest mit Ritzen oder Pricken der Haut durchzuführen. Hierzu kann in den Behältern 3 jeweils ein im wesentlichen ringförmig ausgebildetes, mit Allergen getränktes Schwammgewebe oder ein gelartiges Allergen derart angeordnet werden, dass es durch Auflegen der Behältereinheit 2 auf die Haut auf letztere übertragbar ist, so dass das Schwammgewebe oder das gelartige Allergen nach Entfernen der Behältereinheit 2 mittels eines Prickwerkzeugs durchstechbar und dadurch das Allergen auf bzw. in die Haut einbringbar ist. Eine als Schablonenteil fungierende Abdeckeinheit erübrigt sich dadurch, da das Anordnungsmuster der Behälter 3 in der Behältereinheit 2 gewissermaßen auf die Haut übertragen wird.

Eine im Rahmen der vorliegenden Erfindung denkbare Abwandlung der Ausführungsform gemäß Fig. 6 besteht darin, sowohl die in Fig. 6 zu sehende Rückseite als auch die in Fig. 6 nicht zu sehende Vorderseite der Vorrichtung 1 jeweils mit einer mittels einer Pricknadel durchstechbaren Folie zu versehen, die jeweils die einzelnen Behälter 3 verschließt. Zum Aufbringen der Allergene auf bzw. in die Haut ist es dann nicht erforderlich, eine der Folien abzuziehen. Stattdessen werden die Behälter 3 nach dem Aufbringen der Vorrichtung 1 auf die Haut lediglich mittels der Pricknadel derart durchstochen, dass in einem einzigen Arbeitsschritt einerseits die Pricknadel mit Allergen benetzt und andererseits die Haut mittels der benetzten Pricknadel geritzt wird. Die so abgewandelte Ausführungsform kann bei Bedarf auch ohne die beiden Kennzeichnungseinheiten 6 ausgebildet werden. Alternativ kann dann auf der in Fig. 6 nicht zu sehenden Vorderseite der Vorrichtung 1 beispielsweise eine in der Art eines Abziehbildes auf die Haut übertragbare Zuordnungsinformation zur Zuordnung bestimmter Allergene zu bestimmten Allergieteststellen angebracht werden.

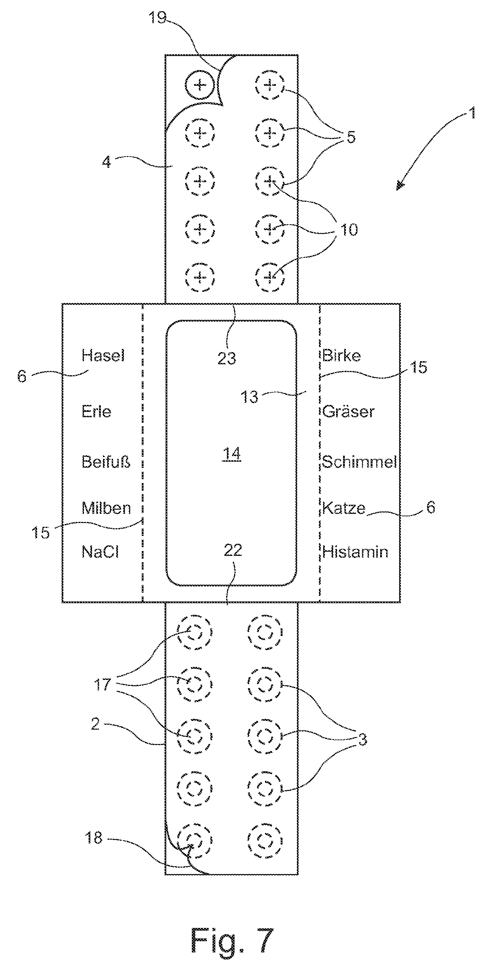

Fig. 7 zeigt eine dritte Ausführungsform einer erfindungsgemäßen Vorrichtung 1. Im Gegensatz zu der ersten Ausführungsform weist die dritte Ausführungsform ein flaches, im Wesentlichen rechteckförmiges Rahmenteil 13 auf, das eine Rahmenöffnung 14 umschließt. An den beiden gegenüberliegenden, langen Seiten des Rahmenteils 13 ist jeweils über eine perforierte Reißlinie 15 eine erfindungsgemäße Kennzeichnungseinheit 6 angeordnet. An der in Fig. 7 unteren, kurzen Seite des Rahmenteils 13 ist mittels eines Gelenks 22 eine Behältereinheit 2 befestigt, die ihrem Aufbau nach derjenigen entspricht, die in Zusammenhang mit der ersten Ausführungsform beschrieben wurde. An der in Fig. 7 oberen, kurzen Seite des Rahmenteils 13 ist mittels eines Gelenks 23 eine Abdeckeinheit 4 befestigt, die derjenigen entspricht, die in Zusammenhang mit der ersten Ausführungsform beschrieben wurde.

Nachfolgend werden beispielhaft die einzelnen Arbeitsschritte zur Durchführung eines Allergietests mittels der Ausführungsform gemäß Fig. 7 beschrieben:

Zunächst werden die Schutzfolien 8 von den in Fig. 7 nicht zu sehenden Seiten der beiden Kennzeichnungseinheiten 6 entfernt. Mit Hilfe der dadurch freigelegten Klebefilme wird die Vorrichtung 1 auf der Hautoberfläche, beispielsweise einem Unterarm, einer Testperson befestigt. Anschließend wird die Abziehfolie 19 von der Abdeckeinheit 4 entfernt und letztere um im Wesentlichen 180° um das Gelenk 23 in die Rahmenöffnung 14 geklappt. Dabei sorgt der von der Abziehfolie 19 zuvor bedeckte Klebefilm an der Abdeckeinheit 4 für einen sicheren Halt derselben auf der Hautoberfläche. Alternativ ist denkbar, hier die Abziehfolie 19 einschließlich den von ihr abgedeckten Klebefilm an der Abdeckeinheit 4 ersatzlos weg zu lassen, da ein gewisser Halt der Vorrichtung 1 auf der Hautoberfläche bereits aufgrund der aufgeklebten Kennzeichnungseinheiten 6 gegeben ist. Nach dem Auflegen der Abdeckeinheit 4 auf die Haut erfolgt das Ritzen bzw. Pricken der einzelnen Allergieteststellen in derselben Weise, wie sie in Zusammenhang mit der ersten Ausführungsform beschrieben wurde. In Fig. 7 sind durch die Durchbrüche 5 hindurch die hier kreuzförmig ausgebildeten Durchgangslöcher 10 in der Abziehfolie 9 zu erkennen. Wie voranstehend beschrieben, wird dadurch die Zuverlässigkeit des Hautritzens im Kreuzschnittpunkt erfüllt.

Nach dem Ritzen der Haut wird die in Fig. 7 nicht zu erkennende Abziehfolie 9 abgezogen, so dass die Durchbrüche 5 vollständig freiliegen. Des Weiteren wird zum Öffnen der Behälter 3 die Schließfolie 18 von der Behältereinheit 2 entfernt. Daraufhin kann die Behältereinheit 2 mit den offenen Behältern 3 um im Wesentlichen 180° um das Gelenk 22 in die Rahmenöffnung 14 und damit auf die Abdeckeinheit 4 geklappt werden. Der von der Abziehfolie 9 zuvor bedeckte Klebefilm an der Abdeckeinheit 4 sorgt dabei für einen sicheren Halt der Behältereinheit 2 an der Abdeckeinheit 4. Es entsteht eine kompakt handhabbare Einheit umfassend das Rahmenteil 13, die Abdeckeinheit 4 und die Behältereinheit 2. Nach einer gewissen Einwirkdauer kann diese kompakt handhabbare Einheit vollständig von der Hautoberfläche entfernt werden, indem die Kennzeichnungseinheiten 6 niedergedrückt oder festgehalten werden und die kompakt handhabbare Einheit entlang der beiden perforierten Reißlinien 15 abgerissen wird.

### BEZUGSZEICHENLISTE

- 1: Vorrichtung
- 2: Behältereinheit
- 3: Behälter
- 4: Abdeckeinheit
- 5: Durchbruch
- 6: Kennzeichnungseinheit
- 7: Reißlinie
- 8: Schutzfolie
- 9: Abziehfolie
- 10: Durchgangslöcher
- 11: Kreismittelpunkt
- 12: Dichtlippe
- 13: Rahmenteil
- 14: Rahmenöffnung
- 15: Reißlinie
- 16: Gelenk
- 17: Schwammgewebe
- 18: Schließfolie
- 19: Abziehfolie
- 20: Durchgangsöffnungen
- 21: Bereich der Abziehfolie 9
- 22, 23: Gelen

## Patentansprüche

1. Vorrichtung zur Durchführung eines Allergietests, umfassend eine Behältereinheit (2) mit mehreren Behältern (3) zur Aufnahme von Allergenen, wobei die Vorrichtung eine auf die Haut übergabefähige Kennzeichnung zur Zuordnung von bestimmten Allergenen zu bestimmten Allergieteststellen auf der Haut eines Testlebewesens aufweist,
**dadurch gekennzeichnet, dass**
die übergabefähige Kennzeichnung von wenigstens einer Kennzeichnungseinheit (6) gebildet wird, die lösbar an der Vorrichtung befestigt und auf ihrer bei Durchführung des Allergietests der Haut zugewandten Seite zumindest teilweise mit einem Haftmittel versehen ist, so dass die Vorrichtung bei auf der Haut verbleibender Kennzeichnungseinheit (6) entfernbar ist.

2. Vorrichtung nach Anspruch 1,
**dadurch gekennzeichnet, dass**
sie eine Abdeckeinheit (4) mit mehreren Durchbrüchen (5) zur Abgrenzung der Allergieteststellen aufweist, wobei die Abdeckeinheit (4) auf die Haut des Testlebewesens auflegbar ist und die Behältereinheit (2) derart auf die Abdeckeinheit (4) auflegbar ist, dass die Durchbrüche (5) mit den Behältern (3) im Wesentlichen fluchten.

3. Vorrichtung nach Anspruch 1 oder 2,
**dadurch gekennzeichnet, dass**
die Kennzeichnungseinheit (6) lösbar an einer Seitenkante der Behältereinheit (2) oder der Abdeckeinheit (4) befestigt ist, so dass die Behältereinheit (2) und/oder die Abdeckeinheit (4) bei auf der Haut verbleibender Kennzeichnungseinheit (6) entfernbar ist/sind.

4. Vorrichtung nach Anspruch 3,
**dadurch gekennzeichnet, dass**
die wenigstens eine Kennzeichnungseinheit (6) entlang einer perforierten Reißlinie (7) an der Behältereinheit (2) oder der Abdeckeinheit (4) befestigt ist.

5. Vorrichtung nach einem der vorangehenden Ansprüche,
**dadurch gekennzeichnet, dass**
die wenigstens eine Kennzeichnungseinheit (6) um im Wesentlichen 180° zwischen einer ersten Stellung, in der sie auf der Behältereinheit (2) oder der Abdeckeinheit (4) aufliegt, und einer zweiten Stellung, in der sie von der Seitenkante der Behältereinheit (2) oder der Abdeckeinheit (4) abragt, schwenkbar ist.

6. Vorrichtung nach einem der vorangehenden Ansprüche,
**dadurch gekennzeichnet, dass**
an zwei gegenüberliegenden Seitenkanten der Behältereinheit (2) oder der Abdeckeinheit (4) jeweils eine Kennzeichnungseinheit (6) angeordnet ist.

7. Vorrichtung nach einem der vorangehenden Ansprüche,
**dadurch gekennzeichnet, dass**
die Behälter (3) aus der Behältereinheit (2) herauslösbar sind, so dass die Behältereinheit (2) oder die Behältereinheit (2) und die Abdeckeinheit (4) bei auf der Haut verbleibenden Behältern (3) entfernbar ist.

8. Vorrichtung nach Anspruch 2,
**dadurch gekennzeichnet, dass**
ein Rahmenteil (13) mit einer Rahmenöffnung (14) vorgesehen ist, wobei die Behältereinheit (2), die Abdeckeinheit (4) und die wenigstens eine Kennzeichnungseinheit (6) jeweils an einer äußeren Seitenkante des Rahmenteils (13) befestigt sind.

9. Vorrichtung nach Anspruch 8,
**dadurch gekennzeichnet, dass**
die Behältereinheit (2) und die Abdeckeinheit (4) gelenkig an dem Rahmenteil (13) befestigt sind, so dass sie um im wesentlichen 180° in die Rahmenöffnung (14) klappbar sind.

10. Vorrichtung nach Anspruch 8 oder 9,
**dadurch gekennzeichnet, dass**
die wenigstens eine Kennzeichnungseinheit (6) entlang einer perforierten Reißlinie (15) an dem Rahmenteil (13) befestigt ist.

11. Vorrichtung nach einem der Ansprüche 8 bis 10,
**dadurch gekennzeichnet, dass**
die Behältereinheit (2) und die Abdeckeinheit (4) an gegenüberliegenden Seiten des Rahmenteils (13) befestigt sind.

12. Vorrichtung nach einem der Ansprüche 8 bis 11,
**dadurch gekennzeichnet, dass**
die wenigstens eine Kennzeichnungseinheit (6) um im Wesentlichen 180° zwischen einer ersten Stellung, in der sie im Wesentlichen in der Rahmenöffnung (14) liegt, und einer zweiten Stellung, in der sie von der Seitenkante des Rahmenteils (13) abragt, schwenkbar ist.

13. Vorrichtung nach einem der Ansprüche 8 bis 12,
**dadurch gekennzeichnet, dass**
an zwei gegenüberliegenden Seitenkanten des Rahmenteils (13) jeweils eine Kennzeichnungseinheit (6) angeordnet ist.

14. Vorrichtung nach einem der vorangehenden Ansprüche,
**dadurch gekennzeichnet, dass**
das Haftmittel ein von einer entfernbaren Schutzfolie (8) bedeckter Klebefilm ist.

15. Vorrichtung nach einem der vorangehenden Ansprüche,
**dadurch gekennzeichnet, dass**
in den Behältern (3) jeweils im Wesentlichen ringförmig ausgebildetes, mit Allergen getränktes Schwammgewebe oder gelartiges Allergen derart angeordnet ist, dass es durch Auflegen der Behältereinheit (2) auf die Haut auf letztere übertragbar ist, so dass das Schwammgewebe oder das gelartige Allergen nach Entfernen der Behältereinheit (2) mittels eines Prickwerkzeugs durchstechbar und **dadurch** das Allergen in die Haut einbringbar ist.
